# EUROPEAN PATENT APPLICATION

(11) **EP 3 721 802 A1**
(43) Date of publication of application: **14.10.2020**
(21) Application number: 19167845.7
(22) Date of filing: 08.04.2019
(51) Int. Cl.: A61B 5/145, A61B 5/1495, A61B 5/1486

(54) **MICRODIALYSIS SYSTEM**

(71) Applicant: M Dialysis AB, 121 05 Stockholm (SE)
(72) Inventor: NORD, Olof, 132 46 Saltsjö-Boo (SE)
(74) Representative: AWA Sweden AB

(57) **Abstract**

A microdialysis system (100) is disclosed, comprising a microdialysis probe (30) insertable or inserted into tissue of a subject (40), a sensor module (50) configured to receive at least one fluid flow and sense at least one substance in the received at least one fluid flow, at least one fluid flow transport device configured to selectively convey a flow of fluid to the microdialysis probe (30), and at least one fluid flow path (60) for flow of fluid to the sensor module (50). The at least one fluid flow transport device is configured to selectively convey a flow of fluid including at least one reagent so as to join with the at least one fluid flow path (60) for flow of fluid to the sensor module (50) via at least one fluid flow connector (65; 70) for joining flow of fluid including at least one reagent with the at least one fluid flow path (60) for flow of fluid to the sensor module (50). The at least one fluid flow transport device is further configured to selectively convey a flow of calibration fluid so as to join with the at least one fluid flow path (60) for flow of fluid to the sensor module (50) via at least one fluid flow connector (65; 80) for joining flow of calibration fluid with the at least one fluid flow path (60) for flow of fluid to the sensor module (50). Each of the at least one fluid flow connector (65; 70, 80) is arranged and/or configured so as permit the joining of the respective flows with the at least one fluid flow path (60) for flow of fluid to the sensor module (50) without requiring any moving parts, e.g., by being implemented as one or more valveless fluid flow connectors.

## Description

### TECHNICAL FIELD

The present invention relates to a microdialysis system.

### BACKGROUND

Many diagnostic and therapeutic decisions in clinical practice are based on measuring substances in blood, even though most biochemical and pharmacological events take place in individual tissues and organs. So called microdialysis offers a possibility to monitor tissue and organ chemistry, and is rapidly making its way into clinical practice and medical research. Microdialysis enables monitoring of a chemical event taking place in an interstitial fluid. Samples can be collected and analyzed repeatedly to get trends of in-vivo tissue chemical changes. An advantage of microdialysis is the possibility to detect early signs of tissue ischemia. When the supply of glucose and oxygen is inadequate, it is followed by an increase in the ratio of Lactate and Pyruvate (LP-ratio), which is a known marker for tissue ischemia. In microdialysis, a relatively thin catheter is introduced into the tissue of interest in a subject (e.g., the brain tissue of the subject), and a pump continuously perfuses the interior of the catheter with a physiological fluid. The physiological fluid may comprise an aqueous solution (perfusate) that closely resembles the (ionic) composition of the surrounding tissue fluid. The interior of the catheter is in general continuously perfused at a relatively low flow rate of about 0.1-5 µl per minute. A semi-permeable membrane at the distal end of the catheter may function like a blood capillary, and chemical substances from the interstitial fluid may diffuse across the membrane into the perfusion fluid, which perfusion fluid may be continuously collected in micro vials and analyzed in an analyzer as often as needed. The results of the analysis can then be displayed to the user, e.g., on a display. The physiological fluid, with which the interior of the catheter is continuously perfused, is conveyed from the catheter directly to the analyzer, or possibly via the microvial.

### SUMMARY

In at least some microdialysis applications it may be desirable to be able to carry out a microdialysis operation over an extended, continuous period of time, over which period of time flow of fluid through the different parts of the microdialysis system is maintained. For such microdialysis applications, it would be desirable to use equipment that require less maintenance (e.g., less cleaning) and which are less prone to being clogged up by fluid flow, in which case the microdialysis operation may have to be interrupted for cleaning, etc.

In view of the foregoing, a concern of the present invention is to provide a microdialysis system that may be made relatively simple and robust, and possibly relatively easy to maintain.

To address at least one of this concern and other concerns, a system in accordance with the independent claim is provided. Preferred embodiments are defined by the dependent claims.

According to an aspect of the present invention there is provided a microdialysis system, which comprises a microdialysis probe that is insertable or inserted into tissue of a subject. The microdialysis probe is configured so that it can receive a fluid flow and so that it can output a fluid flow. The microdialysis system comprises a sensor module, which is configured to receive at least one fluid flow and sense at least one substance in the received at least one fluid flow. The microdialysis system comprises at least one fluid flow transport device configured to selectively convey a flow of fluid to the microdialysis probe. The microdialysis system comprises at least one fluid flow path for flow of fluid to the sensor module. The at least one fluid flow transport device is configured to convey a flow of fluid having exited the microdialysis probe (which flow of fluid may be referred to as a sample fluid flow) into, or so as to join with, the at least one fluid flow path (or possibly several fluid flow paths) for flow of fluid to the sensor module. The at least one fluid flow transport device is configured to selectively convey a flow of fluid including at least one reagent so as to join with the at least one fluid flow path for flow of fluid to the sensor module via at least one fluid flow connector for joining flow of fluid including at least one reagent with the at least one fluid flow path for flow of fluid to the sensor module (which may possibly be referred to as at least one first fluid flow connector). The at least one fluid flow transport device is configured to selectively convey a flow of calibration fluid so as to join with the at least one fluid flow path for flow of fluid to the sensor module via at least one fluid flow connector for joining flow of calibration fluid with the at least one fluid flow path for flow of fluid to the sensor module (which may possibly be referred to as at least one second fluid flow connector). Each of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module is configured (and/or arranged) so as to permit joining of the flow of fluid including at least one reagent and the flow of calibration fluid, respectively, with the at least one fluid flow path for flow of fluid to the sensor module without requiring any moving parts (i.e., without requiring any moving parts of or in the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module).

Thus, the flow of fluid including at least one reagent and the flow of calibration fluid, respectively, may be joined with the at least one fluid flow path for flow of fluid downstream the microdialysis probe and upstream the sensor module. The at least one fluid flow path for flow of fluid to the sensor module may extend between the microdialysis probe and the sensor module, and may (possibly selectively) receive a flow of fluid having exited the microdialysis probe, selectively receive a flow of fluid including at least one reagent, and selectively receive a flow of calibration fluid, for further conveyance to the sensor module. By way of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module, the flow of fluid including at least one reagent and the flow of calibration fluid, respectively, may be joined with the at least one fluid flow path for flow of fluid to the sensor module possibly without or with less need for moving parts, e.g., in devices such as valves, for realizing the joining of flows. The conveying of the respective ones of the flow of fluid including at least one reagent and the flow of calibration fluid so as to join with the at least one fluid flow path for flow of fluid to the sensor module may hence take place without requiring or at least with less need of valves or other devices with moving parts for implementing the joining of the respective ones of the flow of fluid including at least one reagent and the flow of calibration fluid with the at least one fluid flow path for flow of fluid to the sensor module. This entails that the construction of the at least one fluid flow transport device and/or other components of the microdialysis system effecting the above-mentioned joining of the flows may be made relatively simple and robust, and possibly easier to maintain, since for example less or even no cleaning of valves or other devices with moving parts for effecting the above-mentioned joining of the flows may be required.

The microdialysis probe may be configured so that it can receive a flow of physiological fluid and so that it can output a flow of physiological fluid. The at least one fluid flow transport device may be configured to convey a flow of physiological fluid to the microdialysis probe, and convey a flow of physiological fluid having exited the microdialysis probe into, or so as to join with, the at least one fluid flow path for flow of fluid to the sensor module. The physiological fluid may for example comprise an aqueous solution (perfusate) that closely resembles the (ionic) composition of the surrounding tissue fluid of the tissue of the subject.

The microdialysis probe may for example comprise or be constituted by a microdialysis catheter. In the context of the present application, by a microdialysis probe, or a microdialysis catheter, it is meant a device that includes a catheter that is designed so as to mimic in terms of functionality a blood capillary, and which may comprise a tubular structure such as a shaft having a semipermeable membrane at a distal end thereof. The interior of the catheter may be continuously perfused with a physiological fluid that may comprise an aqueous solution (perfusate) that resembles the (ionic) composition of the fluid of surrounding tissue in which the catheter is inserted. Once the catheter is inserted into the tissue of interest, chemical substances from the interstitial fluid may cross the semipermeable membrane by means of passive diffusion. The physiological fluid leaving the catheter may be referred to as dialysate.

Thus, the fluid flow (e.g., of a physiological fluid) received by the microdialysis probe may generally be referred to as perfusate, and the fluid flow output by the microdialysis probe may generally be referred to as dialysate.

The subject may for example be a human subject. The tissue of the subject may for example be constituted by or comprise brain tissue of the subject, but is not limited thereto, and may, in addition or in alternative, comprise or be constituted by tissue of the subject other than brain tissue.

The microdialysis system may comprise at least one fluid flow passage, or fluid flow conduit (e.g., comprising tubing and/or piping, which for example may be made of plastic or another suitable material), for conveying of the fluid (e.g., a physiological fluid) having exited the microdialysis probe to the sensor module, e.g., into or so as to join with the at least one fluid flow path for flow of fluid to the sensor module. The at least one fluid flow path for flow of fluid to the sensor module may comprise or be constituted by the at least one fluid flow conduit. The at least one fluid flow conduit may comprise the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module. Each of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module may be arranged downstream the microdialysis probe and upstream the sensor module. The at least one fluid flow transport device may be configured to selectively convey the flow of fluid including at least one reagent into the at least one fluid flow conduit (e.g., so as to join with the flow of fluid having exited the microdialysis probe conveyed in the at least one fluid flow conduit) via the at least one fluid flow connector for joining flow of fluid including at least one reagent with the at least one fluid flow path for flow of fluid to the sensor module. The at least one fluid flow transport device may be configured to selectively convey the flow of calibration fluid into the at least one fluid flow conduit (e.g., so as to join with the flow of fluid having exited the microdialysis probe conveyed in the at least one fluid flow conduit) via the at least one fluid flow connector for joining flow of calibration fluid with the at least one fluid flow path for flow of fluid to the sensor module. The conveying of the respective fluids into the fluid flow conduit may hence take place without requiring valves or other moving parts for implementing the introduction of the respective fluids into the fluid flow conduit, which entails that the construction of the at least one fluid flow conduit can be made relatively simple and robust, and possibly easier to maintain, since less cleaning of the first inlet and the second inlet may be required as compared to if valves or other devices with moving parts would be employed.

At least one of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module may for example comprise or be constituted by a valveless fluid flow connector. At least one of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module may for example comprise or be constituted by a so called Y-coupling, or a so called T-coupling.

The at least one fluid flow transport device may be configured to selectively convey the flow of fluid having exited the microdialysis probe so as to join with the at least one fluid flow path for flow of fluid to the sensor module via at least one fluid flow connector for joining flow of fluid having exited the microdialysis probe with the at least one fluid flow path for flow of fluid to the sensor module (which may possibly be referred to as at least one third fluid flow connector). The at least one fluid flow connector for joining flow of fluid having exited the microdialysis probe with the at least one fluid flow path for flow of fluid to the sensor module may be arranged downstream the microdialysis probe and upstream the sensor module. The at least one fluid flow connector for joining flow of fluid having exited the microdialysis probe with the at least one fluid flow path for flow of fluid to the sensor module may be configured so as to permit joining of the flow of fluid having exited the microdialysis probe with the at least one fluid flow path for flow of fluid to the sensor module without requiring any moving parts. The flow of fluid having exited the microdialysis probe may hence be joined with the at least one fluid flow path for flow of fluid to the sensor module possibly without or with less need for moving parts, e.g., in devices such as valves, for realizing the joining of flow. The conveying of the flow of fluid having exited the microdialysis probe so as to join with the at least one fluid flow path for flow of fluid to the sensor module may hence take place without requiring or at least with less need of valves or other devices with moving parts for implementing the joining of the flow of fluid having exited the microdialysis probe with the at least one fluid flow path for flow of fluid to the sensor module. This entails that the construction of the at least one fluid flow transport device and/or other components of the microdialysis system effecting the above-mentioned joining of the flow may be made relatively simple and robust, and possibly easier to maintain, since for example less or even no cleaning of valves or other devices with moving parts for effecting the above-mentioned joining of the flows may be required.

As already mentioned in the foregoing, the at least one fluid flow path for flow of fluid to the sensor module may comprise at least one fluid flow conduit for conveying of fluid to the sensor module. The at least one fluid flow conduit may comprise the at least one fluid flow connector for joining flow of fluid having exited the microdialysis probe with the at least one fluid flow path for flow of fluid to the sensor module. The at least one fluid flow transport device may be configured to selectively convey the flow of fluid having exited the microdialysis probe into the at least one fluid flow conduit via the at least one fluid flow connector for joining flow of fluid having exited the microdialysis probe with the at least one fluid flow path for flow of fluid to the sensor module.

The at least one fluid flow connector for joining flow of fluid having exited the microdialysis probe with the at least one fluid flow path for flow of fluid to the sensor module may for example comprise a valveless fluid flow connector, e.g., comprising or being constituted by a so called Y-coupling, or a so called T-coupling.

The at least one fluid flow transport device may be configured to convey the flow of fluid having exited the microdialysis probe, the flow of fluid including at least one reagent, and the flow of calibration fluid, and/or to join the respective ones of said fluid flows with the at least one fluid flow path for flow of fluid to the sensor module in different manners, such as in relation to each other and, e.g., with respect to where in the at least one fluid flow path for flow of fluid to the sensor module the respective ones of said fluid flows are joined with the at least one fluid flow path for flow of fluid. This will be described in further detail in the following with reference to exemplifying embodiments of the present invention.

For example, the at least one fluid flow transport device may be configured to convey the flow of fluid having exited the microdialysis probe so as to join with the at least one fluid flow path for flow of fluid to the sensor module at a position in the at least one fluid flow path for flow of fluid to the sensor module that is downstream a position in the at least one fluid flow path for flow of fluid to the sensor module in which the flow of fluid including at least one reagent is joined with the at least one fluid flow path for flow of fluid to the sensor module and/or a position in the at least one fluid flow path for flow of fluid to the sensor module in which the flow of calibration fluid is joined with the at least one fluid flow path for flow of fluid to the sensor module. Thus, in the at least one fluid flow path for flow of fluid to the sensor module, the flow of fluid having exited the microdialysis probe may be introduced downstream with respect to where the flow of fluid including at least one reagent and/or the flow of calibration fluid is or are introduced.

According to another example, the at least one fluid flow transport device may be configured to selectively convey the flow of fluid including at least one reagent so as to join with the at least one fluid flow path for flow of fluid to the sensor module at a position in the at least one fluid flow path for flow of fluid to the sensor module that is downstream a position in the at least one fluid flow path for flow of fluid to the sensor module in which the flow of fluid having exited the microdialysis probe is joined with the at least one fluid flow path for flow of fluid to the sensor module and/or a position in the at least one fluid flow path for flow of fluid to the sensor module in which the flow of calibration fluid is joined with the at least one fluid flow path for flow of fluid to the sensor module. Thus, in the at least one fluid flow path for flow of fluid to the sensor module, the flow of fluid including at least one reagent may be introduced downstream with respect to where the flow of fluid having exited the microdialysis probe and/or the flow of calibration fluid is or are introduced.

According to another example, the at least one fluid flow transport device may be configured to selectively convey the flow of calibration fluid so as to join with the at least one fluid flow path for flow of fluid to the sensor module that is downstream a position in the at least one fluid flow path for flow of fluid to the sensor module in which the flow of fluid having exited the microdialysis probe is joined with the at least one fluid flow path for flow of fluid to the sensor module and/or a position in the at least one fluid flow path for flow of fluid to the sensor module in which the flow of fluid including at least one reagent is/are joined with the at least one fluid flow path for flow of fluid to the sensor module is joined with the at least one fluid flow path for flow of fluid to the sensor module. Thus, in the at least one fluid flow path for flow of fluid to the sensor module, the flow of calibration fluid may be introduced downstream with respect to where the flow of fluid having exited the microdialysis probe and/or the flow of fluid including at least one reagent is or are introduced.

In addition to or in alternative, the at least one fluid flow transport device may be configured to join two of: the flow of fluid having exited the microdialysis probe, the flow of fluid including at least one reagent, and the flow of calibration fluid, so as to obtain a combined fluid flow. Any two of the flow of fluid having exited the microdialysis probe, the flow of fluid including at least one reagent, and the flow of calibration fluid, may be joined so as to obtain the combined fluid flow. The at least one fluid flow transport device may be configured to join the third (or remaining) one of the flow of fluid having exited the microdialysis probe, the flow of fluid including at least one reagent, and the flow of calibration fluid (i.e. the remaining fluid flow not in the said combined fluid flow), with the combined fluid flow, e.g., at a position in the at least one fluid flow path for flow of fluid to the sensor module that is downstream the microdialysis probe. The at least one fluid flow transport device may be configured to join the third one of the flow of fluid having exited the microdialysis probe, the flow of fluid including at least one reagent, and the flow of calibration fluid with the combined fluid flow via at least one fluid flow connector (e.g., at least one fourth fluid flow connector), which at least one fluid flow connector may be configured (and/or arranged) so as to permit joining of the flows of fluid without requiring any moving parts (i.e., without requiring any moving parts of or in the at least one (fourth) fluid flow connector).

The least one fluid flow transport device may be controllable with respect to operation thereof.

The least one fluid flow transport device may for example be configured to controllably convey the flow of fluid (e.g., a physiological fluid) to the microdialysis probe and convey the flow of fluid (e.g., a physiological fluid) having exited the microdialysis probe to the sensor module, at least with respect to a flow rate of the flow of fluid to the microdialysis probe and a flow rate of the flow of fluid having exited the microdialysis probe to the sensor module, respectively.

The microdialysis system may comprise at least one control module, which in alternative may be referred to as at least one control unit, or at least one controller.

The at least one control module may be configured to control operation of the at least one fluid flow transport device at least so as to convey the flow of fluid (e.g., a physiological fluid) to the microdialysis probe and convey the flow of fluid (e.g., a physiological fluid) having exited the microdialysis probe to the sensor module so as to maintain a continuous (e.g., constant) flow of fluid to the microdialysis probe and a continuous (e.g., constant) flow of fluid having exited the microdialysis probe to the sensor module over a selected period of time.

The selected period of time may for example be one or several hours, or one or several days and nights (i.e. one or more twenty-four hour periods), e.g., one, two, three, four or five days and nights, or more. Thus, the microdialysis system may be capable of carrying out a continuous microdialysis operation on a subject over an extended period of time.

The at least one fluid flow transport device may be configured to controllably convey the flow of fluid including at least one reagent so as to join with the at least one fluid flow path for flow of fluid to the sensor module, e.g., by controllably conveying the flow of fluid including at least one reagent into the at least one fluid flow conduit, at least with respect to a flow rate of the flow of fluid including at least one reagent.

The at least one control module may be configured to control operation of the at least one fluid flow transport device at least so as to convey the flow of fluid including at least one reagent so as to join with the at least one fluid flow path for flow of fluid to the sensor module, e.g., by controllably conveying the flow of fluid including at least one reagent into the at least one fluid flow conduit, so as to maintain a continuous (e.g., constant) flow of the fluid including at least one reagent over said selected period of time or over another period of time.

The other period of time may be at least in part overlapping with the said selected period of time.

The at least one fluid flow transport device may be configured to controllably convey the flow of calibration fluid so as to join with the at least one fluid flow path for flow of fluid to the sensor module, e.g., by controllably conveying the flow of calibration fluid into the at least one fluid flow conduit, at least with respect to a flow rate of the flow of calibration fluid.

The at least one fluid flow transport device may be configured to selectively either convey a flow of fluid to the microdialysis probe and convey a flow of fluid having exited the microdialysis probe to the sensor module via the at least one fluid flow path for flow of fluid to the sensor module, or convey a flow of calibration fluid so as to join with the fluid flow path for flow of fluid having exited the microdialysis probe to the sensor module via the at least one fluid flow connector for joining flow of calibration fluid with the at least one fluid flow path for flow of fluid to the sensor module, but not concurrently convey a flow of fluid to the microdialysis probe and convey a flow of fluid having exited the microdialysis probe to the sensor module via the at least one fluid flow path for flow of fluid to the sensor module, and convey a flow of calibration fluid so as to join with the at least one fluid flow path for flow of fluid to the sensor module via the at least one fluid flow connector for joining flow of calibration fluid with the at least one fluid flow path for flow of fluid to the sensor module.

For example, the at least one fluid flow transport device may be configured to selectively either convey the flow of calibration fluid into the at least one fluid flow conduit or convey a flow of fluid having exited the microdialysis probe into the at least one fluid flow conduit, but not concurrently convey both the flow of calibration fluid and the flow of fluid having exited the microdialysis probe into the at least one fluid flow conduit.

A calibration process for, e.g., the sensor module, may be carried out.

The at least one fluid flow transport device may be configured to selectively convey a flow of calibration fluid so as to join with the at least one fluid flow path for flow of fluid to the sensor module via the at least one fluid flow connector for joining flow of calibration fluid with the at least one fluid flow path for flow of fluid to the sensor module while not conveying a flow of fluid to the microdialysis probe. Thereby, there may not be conveyed a flow of fluid having exited the microdialysis probe to the sensor module via the at least one fluid flow path for flow of fluid to the sensor module. This may be done in order to carry out a calibration process for the sensor module, and may be done based on at least one predefined trigger criterion (e.g., may be done if at least one trigger criterion is met). The at least one predefined trigger criterion may for example comprise at least one of: that a selected period of time of use of the microdialysis system has elapsed, or that a selected period of time since the carrying out of a previous calibration process has elapsed.

The calibration process for the sensor module may be carried out automatically, for example on a condition that at least one of the at least one predefined trigger criterion is met. To that end, the microdialysis system may comprise at least one control module (which may be same as the at least one control module described in the foregoing) configured to control operation of the at least one fluid flow transport device at least such that the calibration process for the sensor module is automatically carried out on a condition that at least one of the at least one predefined trigger criterion is met.

The at least one fluid flow transport device may at least in part comprise the at least one fluid flow conduit. In alternative or in addition, the sensor module may at least in part comprise the at least one fluid flow conduit.

According to one or more embodiments of the present invention, the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module may be the same.

According to one or more other embodiments of the present invention, the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module may be different. The at least one fluid flow connector for joining flow of calibration fluid with the at least one fluid flow path for flow of fluid to the sensor module may be downstream the at least one fluid flow connector for joining flow of fluid including at least one reagent with the at least one fluid flow path for flow of fluid to the sensor module. In alternative, the at least one fluid flow connector for joining flow of fluid including at least one reagent with the at least one fluid flow path for flow of fluid to the sensor module may be downstream the at least one fluid flow connector for joining flow of calibration fluid with the at least one fluid flow path for flow of fluid to the sensor module.

The sensor module may be (further) configured so that it can output the at least one fluid flow after sensing of the at least one substance in the at least one fluid flow.

The sensor module may for example comprise a so called flow-through sensor. The sensor module may be arranged at a relatively small distance from the microdialysis probe. The sensor module could possibly be arranged in close proximity to the subject or even be attached to the subject. For example, the sensor module may be arranged so as to wearable by the subject. The microdialysis probe and the sensor module may be in fluid communication with each other, and the fluid conveyed to the sensor module may enter the sensor module.

The sensor module may for example be constituted by or comprise an electrochemical sensor. The sensor module may for example be configured to employ amperometry for sensing the concentration of selected chemical species, e.g., glucose, lactate, and/or pyruvate, in the (physiological) fluid that has exited the microdialysis probe. As mentioned in the foregoing, a semi-permeable membrane at the distal end of the microdialysis probe (e.g., the catheter thereof) may function like a blood capillary, and chemical substances (such as, for example, glucose, lactate, and/or pyruvate) from the interstitial fluid may diffuse across the membrane into the (physiological) fluid. For example, the sensor module may comprise one or more electrodes at which selected chemical substances may oxidize, hence generating a current. The current thereby generated may be proportional to the amount of the chemical substances, or other chemical substances indicative of the selected chemical substances, present in fluid flow, e.g., a physiological fluid flow, received by the sensor module. The current thereby generated may depend on the flow rate of the (physiological) fluid.

As mentioned, the sensor module may generally function similar to an electrochemical sensor and/or an amperometric sensor. By amperometry it is meant sensing of substances in a solution based on electric current or changes in electric current. An electrochemical sensor is a sensor whose working principle is based on production of a current when a potential is applied between two electrodes. The current generated may be proportional to the concentration of chemical species in solution, or in gel (e.g., hydrogel). An amperometric sensor is a sensor that whose working principle is based on measuring current at a constant potential. The sensor module may in alternative, or in addition, be based on one or more dynamic voltammetric technologies, including Chronoamperometry, cyclic voltammetry and differential pulse voltammetry.

For example, the sensor module could include a fluid flow channel block or chip including one or more microfluidic channels in which the (physiological) fluid that has exited the microdialysis probe (e.g., the catheter thereof) may flow. The sensing operation by the sensor module could for example be carried out by means of so called needle electrodes which may be inserted manually by a user in part into one or more of the microfluidic channels in the fluid flow channel block or chip via holes therein extending between a surface of the fluid flow channel block or chip and a lateral wall of the one or more of the microfluidic channels.

The sensor module may be referred to as a biosensor (module), particularly for the sensing of glucose, lactate or pyruvate. In the context of the present application, by a biosensor it is meant a sensor that comprises a biological molecule for sensing or detection an agent, metabolite or molecule. The biological molecule can be any biological molecule, for example can be a protein, for example an antibody or an enzyme, or could be a nucleic acid, for example DNA or mRNA.

The sensor module may be used in, for example, the diagnosis of a medical condition, monitoring the progression of evolving pathology (e.g. traumatic brain injury) or surgical interventions, or for the evaluation of the appropriate treatment regimens. The sensor module may also be used during exercise, for example to aid an athlete in his/her training. The sensor module may for example be used in conjunction with fluid from a human or animal subject, which fluid may be extracellular fluid extracted from the subject by means of microdialysis.

The sensor module may be configured to sense presence and/or concentration of the at least one substance in the received at least one fluid flow (i.e. in the fluid flow received by the sensor module).

The sensor module may be configured to directly sense at least one substance in the received at least one fluid flow, or indirectly sense at least one substance in the received at least one fluid flow. The sensor module may be configured to indirectly sense at least one substance in the received at least one fluid flow for example by sensing at least one first substance in the received at least one fluid flow, and based on the sensing of the at least one first substance, sense at least one second substance. The at least one first substance may be indicative of the at least one second substance. The sensing of the at least one second substance may hence be derived on the sensing of the at least one first substance.

For example, the sensor module may be configured to sense at least one of glucose, lactate, or pyruvate.

The reagent may for example include or be constituted by a co-substrate to an enzymatic reaction or another reaction, for example, one or more of phosphate or phosphoric acid. In alternative or in addition, the reagent may for example include or be constituted by one or more enzymes including, for example, lactate oxidase, glucose oxidase, or pyruvate oxidase, in order to facilitate or allow for sensing (e.g., presence or concentration) of lactate, glucose, or pyruvate, respectively. The reagent is however not limited thereto.

In the context of the present application, by the term reagent it is meant a substance, compound, etc., that is adapted to chemically react with the at least one substance that the sensor module is configured to sense in the at least one fluid flow received by the sensor module in such a way that the sensing of the at least one substance is facilitated for the sensor module, i.e. so that the at least one substance may be easier sensed by the sensor. The reactant may be dissolved in the flow of (physiological) fluid having exited the microdialysis probe to the sensor module (e.g., the dialysate).

For example, in case the sensor module is configured to sense at least pyruvate, the reagent may include phosphate, or phosphoric acid, or pyruvate oxidase. By introducing, e.g., phosphate or phosphoric acid in a flow of physiological fluid that has exited the microdialysis probe and is being conveyed to the sensor module, the phosphate or phosphoric acid may chemically react with any pyruvate in the flow of physiological fluid that has exited the microdialysis probe in such a way that the sensing of any pyruvate by the sensor module in the flow of physiological fluid that has exited the microdialysis probe and that is input into the sensor module may be facilitated. This may be particularly useful for example in case the concentration of phosphate in the flow of physiological fluid that has exited the microdialysis probe is low. For example for subjects suffering from multiple diseases and/or having been hospitalized for a relatively long time, the concentration of phosphate in the blood of the subject may be relatively low and the concentration of pyruvate may be difficult to sense by means of microdialysis unless a reactant is introduced in the flow of physiological fluid that has exited the microdialysis probe and is being conveyed to the sensor module. In view of the foregoing, the sensor module may for example be configured to (directly or indirectly) sense at least pyruvate, wherein the reagent may include phosphate, or phosphoric acid.

The at least one fluid flow transport device may for example comprise at least three fluid transport devices, for example, at least a first fluid flow transport device, a second fluid flow transport device, and a third fluid flow transport device.

The first fluid flow transport device may be configured to convey a flow of a fluid (e.g., a physiological fluid) to the microdialysis probe, and possibly also to convey a flow of a fluid (e.g., a physiological fluid) having exited the microdialysis probe to the sensor module.

The second fluid flow transport device may be configured to selectively (and possibly controllably) convey the flow of fluid including at least one reagent so as to join with the flow of fluid having exited the microdialysis probe to the sensor module, e.g., by selectively (and possibly controllably) conveying the flow of fluid including at least one reagent into the at least one fluid flow conduit.

The third fluid flow transport device may be configured to selectively (and possibly controllably) convey the flow of calibration fluid so as to join with the flow of fluid having exited the microdialysis probe to the sensor module, e.g., by selectively (and possibly controllably) conveying the flow of calibration fluid into the at least one fluid flow conduit.

The at least one fluid flow transport device could comprise another fluid flow transport device (e.g., a fourth fluid transport device) that may be configured to convey a flow of fluid (e.g., physiological fluid) having exited the microdialysis probe to the sensor module (in lieu of the first fluid flow transport device).

The at least one fluid flow transport device may for example comprise at least one pressure pump unit and/or at least one suction pump unit.

The at least one fluid flow transport device may be for example configured to convey a flow of fluid (e.g., physiological fluid) to the microdialysis probe by means of at least one (e.g., first) pressure pump unit (which may be comprised in the at least one fluid flow transport device).

The at least one fluid flow transport device may be configured to selectively (and possibly controllably) convey the flow of fluid including at least one reagent so as to join with the flow of fluid having exited the microdialysis probe to the sensor module, e.g., by selectively (and possibly controllably) conveying the flow of fluid including at least one reagent into the at least one fluid flow conduit, by means of at least one (e.g., second) pressure pump unit (which may be comprised in the at least one fluid flow transport device).

The at least one fluid flow transport device may be configured to selectively (and possibly controllably) convey the flow of calibration fluid so as to join with the flow of fluid having exited the microdialysis probe to the sensor module, e.g., by selectively (and possibly controllably) conveying the flow of calibration fluid into the at least one fluid flow conduit, by means of at least one (e.g., third) pressure pump unit (which may be comprised in the at least one fluid flow transport device).

The at least one fluid flow transport device may be configured to convey a flow of fluid (e.g., physiological fluid) having exited the microdialysis probe to the sensor module by means of at least one suction pump unit (which may be comprised in the at least one fluid flow transport device).

The at least one fluid flow transport device may for example comprise one or more microfluidic flow conduits. The at least one fluid flow transport device may be configured or arranged to provide a steady flow rate of flow of fluid through any fluid flow conduit in the microdialysis system, such as the above-discussed at least one fluid flow conduit, with a flow rate in a range between (about) 0.1 µl per minute (with µl denoting microliter) and (about) 5 µl per minute, or between (about) 0.1 µl per minute and (about) 2 µl per minute, such as, for example, 0.3 µl per minute.

Any fluid flow conduit included in the microdialysis system and/or any pump included in the microdialysis system, e.g., a pressure pump unit and/or a suction pump unit, may for example be made of plastic or a similar material.

Any pump included in the microdialysis system, e.g., a pressure pump unit and/or a suction pump unit, may for example be provided with a piezoelectric motor (also known as a piezo motor) for driving the pump (e.g., effecting the pumping action of the pump).

Further objects and advantages of the present invention are described in the following by means of exemplifying embodiments. It is noted that the present invention relates to all possible combinations of features recited in the claims. Further features of, and advantages with, the present invention will become apparent when studying the appended claims and the description herein. Those skilled in the art realize that different features of the present invention can be combined to create embodiments other than those described herein.

### BRIEF DESCRIPTION OF THE DRAWINGS

Exemplifying embodiments of the invention will be described below with reference to the accompanying drawings.
Figures 1 to 3 are schematic views of microdialysis systems according to one or more embodiments of the present invention.
Figures 4 to 15 schematically illustrate relative arrangements of the flow of fluid having exited the microdialysis probe, the flow of fluid including at least one reagent, and the flow of calibration fluid with respect to the sensor module in accordance with one or more embodiments of the present invention.

The figures are schematic, not necessarily to scale, and generally only show parts which are necessary in order to elucidate embodiments of the present invention, wherein other parts may be omitted or merely suggested.

### DETAILED DESCRIPTION

The present invention will now be described hereinafter with reference to the accompanying drawings, in which exemplifying embodiments of the present invention are illustrated. The present invention may, however, be embodied in many different forms and should not be construed as limited to the embodiments of the present invention set forth herein; rather, these embodiments are provided by way of example so that this disclosure will convey the scope of the present invention to those skilled in the art.

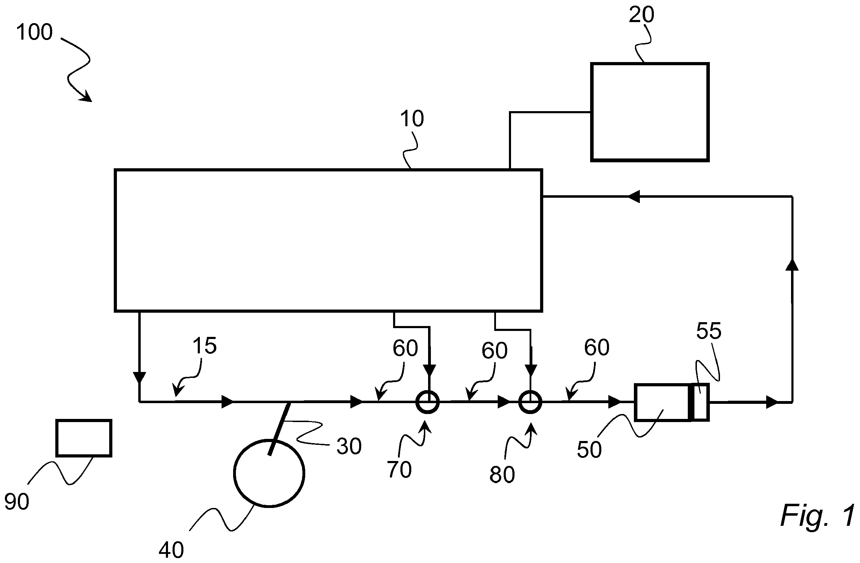

Figure 1 is a (very) schematic view of a microdialysis system 100 according to one or more embodiments of the present invention.

The microdialysis system 100 comprises a microdialysis probe 30, which is insertable or inserted into tissue of a subject, schematically illustrated by reference numeral 40, e.g., brain tissue of the subject 40. The microdialysis probe 30 could in alternative be referred to as a microdialysis catheter. The microdialysis probe 30 is configured so that it can receive a fluid flow, and so that it can output a fluid flow. The subject 40 may for example be a human subject. The tissue of the subject 40 may in addition or in alternative to brain tissue of the subject 40 comprise or be constituted by another or other types of tissue of the subject 40.

The microdialysis system 100 comprises a sensor module 50, which is configured to receive at least one fluid flow. The sensor module 50 is configured to sense at least one substance in the at least one fluid flow received by the sensor module 50. The sensor module 50 may for example be configured to sense presence and/or concentration of the at least one substance in the received at least one fluid flow. The sensor module 50 may for example be configured to sense glucose, lactate, and/or pyruvate, but is not limited thereto.

The microdialysis system 100 comprises at least one fluid flow transport device configured to selectively convey a flow of fluid to the microdialysis probe 30.

The microdialysis system 100 comprises at least one fluid flow path 60 for flow of fluid to the sensor module 50. In accordance with the one or more embodiments of the present invention illustrated in Figure 1, the microdialysis system 100 comprises several interconnected fluid flow paths, indicated in Figure 1 by reference numerals 60, for flow of fluid to the sensor module 50.

In accordance with the one or more embodiments of the present invention illustrated in Figure 1, the microdialysis system 100 comprises an entity 10 which may be referred to as an analyzer, or analysis unit. The analyzer 10 is in fluid communication with the microdialysis probe 30 that is insertable into tissue of the subject 40. Figure 1 very schematically illustrates the situation where the microdialysis probe 30 has been inserted into the subject 40 (e.g., into the brain tissue of the subject 40). In accordance with the one or more embodiments of the present invention illustrated in Figure 1, the at least one fluid flow transport device of the microdialysis system 100 may be included in the analyzer 10. Thus, the at least one fluid flow transport device of the microdialysis system 100 may possibly be referred to by the reference numeral 10. However, it is to be understood that in alternative, or in addition, the at least one fluid flow generator or any other additional fluid flow generator that may be included in the microdialysis system 100 may not be included in the analyzer 10, and may be separately arranged with respect to the analyzer 10.

It is to be understood that Figure 1 is not drawn to scale.

The at least one fluid flow transport device of the microdialysis system 100 is configured to convey a flow of fluid having exited the microdialysis probe 30 into, or so as to join with, the at least one fluid flow path 60 for flow of fluid to the sensor module 50.

The at least one fluid flow transport device of the microdialysis system 100 is configured to selectively convey a flow of fluid including at least one reagent so as to join with the at least one fluid flow path 60 for flow of fluid to the sensor module 50 via a (or at least one) fluid flow connector for joining flow of fluid including at least one reagent with the at least one fluid flow path 60 for flow of fluid to the sensor module 50, schematically indicated at 70 in Figure 1. The fluid flow connector 70 will in the following description be referred to as the first fluid flow connector 70.

The at least one fluid flow transport device of the microdialysis system 100 is configured to selectively convey a flow of calibration fluid so as to join with the at least one fluid flow path 60 for flow of fluid to the sensor module 50 via a (or at least one) fluid flow connector for joining flow of calibration fluid with the at least one fluid flow path for flow of fluid to the sensor module, schematically indicated at 80 in Figure 1. The fluid flow connector 80 will in the following description be referred to as the second fluid flow connector 80.

Each of the first fluid flow connector 70 and the second fluid flow connector 80 is configured so as to permit joining of the flow of fluid including at least one reagent and the flow of calibration fluid, respectively, with the at least one fluid flow path 60 for flow of fluid to the sensor module 50 without requiring any moving parts. To that end, at least one of the first fluid flow connector 70 and the second fluid flow connector 80 may for example comprise a valveless fluid flow connector.

The at least one fluid flow path 60 for flow of fluid to the sensor module 50 may for example comprise at least one fluid flow conduit, which is also referred to be reference numeral 60 (which in alternative may be referred to as at least one fluid flow passage), for conveying of fluid to the sensor module 50. The at least one fluid flow conduit 60 may for example comprise tubing and/or piping, which for example may be made of plastic or another suitable material.

The at least one fluid flow conduit 60 may comprise the first fluid flow connector 70 and the second fluid flow connector 80. For example, the first fluid flow connector 70 and the second fluid flow connector 80 may constitute parts or portions of the at least one fluid flow conduit 60. For example, one or more of the first fluid flow connector 70 and the second fluid flow connector 80 may for example comprise or be constituted by a so called Y-coupling, or a so called T-coupling.

As illustrated in Figure 1, each of the first fluid flow connector 70 and the second fluid flow connector 80 may be arranged downstream the microdialysis probe 30 and upstream the sensor module 50.

The at least one fluid flow transport device of the microdialysis system 100 may be configured to selectively convey the flow of fluid including at least one reagent into the at least one fluid flow conduit 60 via the first fluid flow connector 70. The at least one fluid flow transport device of the microdialysis system 100 may be configured to selectively convey the flow of calibration fluid into the at least one fluid flow conduit 60 via the second fluid flow connector 80.

Any pair of components in the microdialysis system 100, such as the analyzer 10 and the microdialysis probe 30, the microdialysis probe 30 and the sensor module 50, and the sensor module 50 and the analyzer 10, respectively, may be in fluid communication with each other by means of tubing, pipes, conduits, etc. Such fluid communication is indicated in Figure 1 by the lines between the above-mentioned components that are provided with arrows, where the arrows indicate fluid flow direction.

The analyzer 10 may comprise one or more sources of fluid, which for example may comprise a physiological fluid, such as an aqueous solution (which may be referred to as perfusate) that closely resembles the (ionic) composition of the fluid in the surrounding tissue of the microdialysis probe 30 when inserted into the tissue of the subject 40. In alternative, or in addition, the one or more sources of fluid may not be included in the analyzer 10, and may be separately arranged with respect to the analyzer 10. The one or more sources of fluid may for example comprise one or more vials or the like. For example, the analyzer 10 could comprise three sources of fluid, and fluid from each of the sources may be conveyed out of the analyzer 10 by way of separate conduits, such as illustrated in Figure 1 (cf. the arrows between the analyzer 10 and the microdialysis probe 30, the first fluid flow connector 70, and the second fluid flow connector 80, respectively.

The analyzer 10 may comprise sources of fluid for the above-mentioned fluids of the fluid including at least one reagent and the calibration fluid, and for a (or the) physiological fluid described in the foregoing.

The at least one fluid flow transport device of the microdialysis system 100 may for example be configured to selectively convey a flow of fluid to the microdialysis probe 30 via a fluid flow conduit 15, which may possibly be referred to as a perfusate fluid flow conduit 15, or physiological fluid flow conduit 15. As illustrated in Figure 1, the analyzer 10 and the microdialysis probe 30 may be in fluid communication by means of the fluid flow conduit 15. The at least one fluid flow transport device of the microdialysis system 100 may for example be configured to selectively convey a flow of physiological fluid to the microdialysis probe 30 via the fluid flow conduit 15. Thus, a physiological fluid may be conveyed from the analyzer 10 via the fluid flow conduit 15 to the microdialysis probe 30.

As known in the art, the microdialysis probe, or microdialysis catheter, 30 may be designed so as to mimic in terms of functionality a blood capillary, and may for example comprise a tubular structure such as a shaft having a semipermeable membrane at a distal end thereof. The interior of the microdialysis probe 30 may be continuously perfused with a physiological fluid, e.g., a physiological fluid of the type described in the foregoing. When the microdialysis probe 30 is inserted into the tissue of interest in the subject 40, chemical substances from the interstitial fluid may cross the semipermeable membrane by means of passive diffusion. The physiological fluid which subsequently leaves the microdialysis probe 30 may be referred to as an analyte fluid, or dialysate.

The flow of fluid, e.g., a (or the) physiological fluid, which leaves the microdialysis probe 30 may then flow towards sensor module 50 via the at least one fluid flow conduit 60, arranged for flow of fluid to the sensor module 50.

As mentioned in the foregoing, the analyzer 10 may comprise a source of fluid for calibration fluid. During operation of the microdialysis probe 30 the interior of the microdialysis probe (or catheter) 30 may be constantly perfused with physiological fluid that is continuously supplied to the microdialysis probe 30. The sensitivity of the sensor module 50 to chemical substances sensed by or at the sensor module 50 may change over time (e.g., the sensitivity of the sensor module 50 may decrease over time), such as during use of the microdialysis system 100. To that end, a calibration factor, or calibration curve, may be determined. Such calibration factor or calibration curve may for example be determined by means of two calibration fluids, which may be provided from respective sources of calibration fluid, which in accordance with the one or more embodiments of the present invention illustrated in Figure 1 may be arranged in the analyzer 10. The calibration fluids may be conveyed from the analyzer 10 via respective conduits (note however that only one conduit between the analyzer 10 and the first fluid flow connector 70 is illustrated in Figure 1). It is to be understood that more than two calibration fluids could possibly be provided. Each calibration fluid may be provided from a respective one of sources of calibration fluid which may be arranged in the analyzer 10.

By means of the fluid communication between the analyzer 10 (or one or more sources of fluid which may or may not be included in the analyzer 10) and the microdialysis probe 30, physiological fluid may be continuously or continually conveyed or supplied to the microdialysis catheter 30. Thereby, the interior of the microdialysis probe 30 may be continuously or continually perfused with the physiological fluid. The physiological fluid leaving the microdialysis probe 30 (or the dialysate) may then possibly be conveyed back to analyzer 10 (or one or more reservoirs which may or may not be included in the analyzer 10). The physiological fluid collected in the analyzer 10 or reservoir(s) may possibly be analyzed in the analyzer 10 for example in a manner known in the art of microdialysis, and the results of such analysis can be displayed to a user, e.g., on a display, schematically indicated by reference numeral 20 in Figure 1, which display 20 may be connected to the analyzer 10. In alternative, or in addition, the analyzer 10 may be configured to process signals, messages, data, etc., which may be transmitted to the analyzer 10 from the sensor module 50, e.g., for displaying results of the sensing by the sensor module 50 on the display 20. The analyzer 10 may possibly not be configured to (further) analyze physiological fluid, and may possibly only be configured to process signals, messages, data, etc., which may be transmitted to the analyzer 10 from the sensor module 50. As described further in the following, the sensor module 50 may be configured to determine one or more signals that may indicate presence or concentration of different substances which may be present in the fluid flow within the fluid flow conduit 60. The analyzer 10 may be configured to receive those one or more signals from the sensor module 50 and possibly process the signal(s), e.g., for displaying results of the sensing by the sensor module 50 on the display 20. In alternative or in addition to the display 20, some other means for visualizing the results of the sensing by the sensor module 50 and/or the results of the analysis of the physiological fluid may be provided.

For example for effecting the receiving and processing one or more signals from the sensor module 50, the analyzer 10 may for example comprise any suitable central processing unit (CPU), microcontroller, digital signal processor (DSP), Application Specific Integrated Circuit (ASIC), Field Programmable Gate Array (FPGA), etc., or any combination thereof.

As mentioned in the foregoing, the sensor module 50 is configured to sense at least one substance in the at least one fluid flow received by the sensor module 50. The sensor module 50 may for example be configured to sense presence and/or concentration of the at least one substance in the received at least one fluid flow. The sensor module 50 may for example be configured to sense glucose, lactate, and/or pyruvate, but is not limited thereto. The sensor module 50 may for example comprise one or more electrodes at which selected chemical substances may oxidize, hence generating a current, which may be sensed by the sensor module 50. The sensor module 50 may for example comprise sensor module portions (not shown in Figure 1), each of which may be configured to sense presence or concentration of different substances which may be present in the fluid flow within the fluid flow conduit 60, such as, for example, lactate, glucose, or pyruvate, respectively. Each of the sensor module portions may for example comprise one or more electrodes at which selected chemical substances may oxidize, hence generating a (or the) current. The current thereby generated may be proportional to the amount of the chemical substances, or other chemical substances indicative of the selected chemical substances, present in the fluid flow within the fluid flow conduit 60. The sensor module 50 may be configured to determine one or more signals based on the currents thereby generated, which one or more signals may indicate presence or concentration of different substances which may be present in the fluid flow within the fluid flow conduit 60. To that (or some other) end, the sensor module 50 may comprise a processing module or processing unit. The processing module or processing unit may comprise or be constituted by, for example, any suitable central processing unit (CPU), microcontroller, digital signal processor (DSP), Application Specific Integrated Circuit (ASIC), Field Programmable Gate Array (FPGA), etc., or any combination thereof. Possibly, the processing module or processing unit could be arranged separately with respect to sensor module 50 and be connected, e.g., communicatively connected, thereto. The processing module or processing unit of the sensor module 50 may be configured to convert the determined one or more signals for transmission to, e.g., the analyzer 10. For example, the processing module or processing unit of the sensor module 50 may comprise an analog-to-digital converter for converting one or more signals in analog form to digital form.

The above-mentioned calibration factor, or calibration curve, may for example comprise a relationship between the generated current and concentration of different substances which may be present in the fluid flow within the fluid flow conduit 60. By means of the above-mentioned calibration factor, or calibration curve, the amount of the chemical substances, or other chemical substances indicative of the selected chemical substances, present in the fluid flow within the fluid flow conduit 60 may be determined. For example, the amount of the chemical substances, or other chemical substances indicative of the selected chemical substances, present in the fluid flow within the fluid flow conduit 60 may be determined based on the current(s) generated by the sensor module 50 and the above-mentioned calibration factor, or calibration curve.

Values of the current(s) generated by the sensor module 50, or some signal based on the generated current(s), may be transmitted from the sensor module 50 to, e.g., the analyzer 10, possibly for further analysis and/or processing and for displaying the results of the analysis to a user, for example by means of the display 20 and/or some other means for visualizing the results of the analysis. To that end, the sensor module 50 and any entity to which the sensor module 50 communicates the values of the currents thereby generated, or some signal based on the currents thereby generated (e.g., the analyzer 10), may be configured so as to be capable of wired and/or wireless communication, for example using any technique or means for wired and/or wireless communication as known in the art. For example, values of the currents thereby generated, or some signal based on the currents thereby generated, may be transmitted from the sensor module 50 to, e.g., the analyzer 10, continuously or continually. The sensor module 50 could for example comprise a communication module (not shown in any of the figures) which may be capable of wired and/or wireless communication. Similarly, and in alternative or in addition, the analyzer 10 could comprise a communication module (not shown in any of the figures) which may be capable of wired and/or wireless communication.

Since the above-mentioned currents that are generated may be relatively small (for example of the order of 1 nA), transmissions or signaling from the sensor module 50 may be amplified using an amplifier 55, which may be connected to the sensor module 50 and may be configured to amplify the transmissions or signaling from the sensor module 50 so as to facilitate receiving the transmissions or signaling at the entity with which the sensor module 50 may communicate (e.g., the analyzer 10). By amplifying the transmissions or signaling from the sensor module 50, the risk of any disturbance of the transmissions or signaling during transit due to any external influences may be reduced or even avoided. For example, the amplifier 55 may be configured to amplify the one or more signals determined by the sensor module 50 based on the current(s) generated by the sensor module 50. Possibly, the one or more signals determined by the sensor module 50 based on the current(s) generated by the sensor module 50 may be converted in an (or the) analog-to-digital converter, e.g., from analog to digital, before being amplified by the amplifier 55. The amplifier could for example be connected to any communication module comprised in the sensor module 50. Possibly, transmissions or signaling amplifying functionality could be implemented in the communication module. Thus, the amplifier may possibly not be a separate device or element, but could for example be comprised in the communication module.

The sensor module 50 may be arranged at a relatively small distance from the microdialysis probe 30, and may possibly be arranged in close proximity to the subject 40, or may even be attached to (e.g., worn by) the subject 40. In microdialysis applications, the flow rate of the fluid flow within the fluid flow conduit 60 may for example be within a range between (about) 0.1 µl per minute and (about) 5 µl per minute, or between (about) 0.1 µl per minute and (about) 2 µl per minute.

The distance between the subject 40 and the analyzer 10, or the length of the fluid flow path between the subject 40 and the analyzer 10, may be such that it could take about an hour or even more for fluid having exited the microdialysis probe 30 to reach the analyzer 10. Therefore, if the fluid flow (e.g., analyte fluid or dialysate flow) would be analyzed in the analyzer 10 for sensing some quantity, e.g., so as to sense presence or concentration of different substances which may be present in the fluid flow, and not in the sensor module 50, the sensed quantity could possibly only be monitored with a time delay of about one hour or more. However, by the arranging of the sensor module 50 at a relatively small distance from the microdialysis probe 30, and by the sensor module 50 being able to continuously or continually, and in a wired and/or wireless fashion, transmit values of the currents, or some signal based on the currents, generated in the sensing operation of the sensor module 50 as such as described in the foregoing, the sensed quantity could be monitored with less time delay, possibly closer to real time monitoring.

As mentioned in the foregoing, in accordance with the one or more embodiments of the present invention illustrated in Figure 1, the at least one fluid flow transport device of the microdialysis system 100 may be included in the analyzer 10. The at least one fluid flow transport device may in accordance with the one or more embodiments of the present invention illustrated in Figure 1 also be referred to by reference numeral 10, which may be done in the following without any loss of generality. The at least one fluid flow transport device may be provided separately with respect to the analyzer 10.

The at least one fluid flow transport device 10 may for example comprise at least one pressure pump unit and/or at least one suction pump unit.

The at least one fluid flow transport device 10 may be for example configured to convey a flow of fluid (e.g., physiological fluid) to the microdialysis probe 30 by means of at least one first pressure pump unit. The at least one fluid flow transport device 10 may be configured to selectively, and possibly controllably, convey the flow of fluid including at least one reagent so as to join with the flow of fluid having exited the microdialysis probe 30 to the sensor module 50, e.g., by selectively, and possibly controllably, conveying the flow of fluid including at least one reagent into the at least one fluid flow conduit 60, by means of at least one second pressure pump unit. The at least one fluid flow transport device 10 may be configured to selectively, and possibly controllably, convey the flow of calibration fluid so as to join with the flow of fluid having exited the microdialysis probe 30 to the sensor module 50, e.g., by selectively, and possibly controllably, conveying the flow of calibration fluid into the at least one fluid flow conduit 60, by means of at least one third pressure pump unit. The at least one fluid flow transport device 10 may be configured to convey a flow of fluid (e.g., physiological fluid) having exited the microdialysis probe 30 to the sensor module 50 by means of at least one suction pump unit.

For example, the least one fluid flow transport device 10 may for example be configured to controllably convey the flow of fluid (e.g., a physiological fluid) to the microdialysis probe 30 and convey the flow of fluid (e.g., a physiological fluid) having exited the microdialysis probe 30 to the sensor module 50, at least with respect to a flow rate of the flow of fluid to the microdialysis probe 30 and a flow rate of the flow of fluid having exited the microdialysis probe 30 to the sensor module 50, respectively.

In alternative or in addition, the at least one fluid flow transport device 10 may be configured to controllably convey the flow of fluid including at least one reagent so as to join with the at least one fluid flow path for flow of fluid to the sensor module 50, e.g., by controllably conveying the flow of fluid including at least one reagent into the at least one fluid flow conduit 60, at least with respect to a flow rate of the flow of fluid including at least one reagent.

In alternative or in addition, the at least one fluid flow transport device 10 may be configured to controllably convey the flow of calibration fluid so as to join with the at least one fluid flow path for flow of fluid to the sensor module 50, e.g., by controllably conveying the flow of calibration fluid into the at least one fluid flow conduit 60, at least with respect to a flow rate of the flow of calibration fluid.

Each or any of the at least one first pressure pump unit, the at least one second pressure pump unit, the at least one third pressure pump unit, and the at least one suction pump unit, and any other possible pressure pump unit or suction pump unit that may be included in the microdialysis system 100, may be comprised in the at least one fluid flow transport device 10.

The at least one fluid flow transport device may be configured or arranged to provide a steady flow rate of flow of fluid through any fluid flow conduit in the microdialysis system 100, such as any of the above-discussed fluid flow conduits, with a flow rate in a range between (about) 0.1 µl per minute (with µl denoting microliter) and (about) 5 µl per minute, or between (about) 0.1 µl per minute and (about) 2 µl per minute, such as, for example, 0.3 µl per minute.

In accordance with the one or more embodiments of the present invention illustrated in Figure 1, the microdialysis system 100 comprises a control module, schematically indicated by reference numeral 90. The control module 90 may be communicatively coupled with for example the at least one fluid flow transport device 10 and/or any other component in the microdialysis system 100, e.g., for controlling operation thereof. The communicative coupling may be implemented or realized by means of any wired and/or wireless communication means or techniques, for example by means of any wired and/or wireless communication means or techniques as known in the art.

The control module 90 may for example comprise or be constituted by, for example, any suitable CPU, microcontroller, DSP, ASIC, FPGA, etc., or any combination thereof.

The control module 90 may for example be configured to control operation of each or any of the at least one first pressure pump unit, the at least one second pressure pump unit, the at least one third pressure pump unit, and the at least one suction pump unit, and any other possible pressure pump unit or suction pump unit that may be included in the microdialysis system 100, may be comprised in the at least one fluid flow transport device 10.

The control module 90 may be configured to control operation of the at least one fluid flow transport device 10 at least so as to convey the flow of fluid (e.g., a physiological fluid) to the microdialysis probe 30 and convey the flow of fluid (e.g., a physiological fluid) having exited the microdialysis probe 30 to the sensor module 50 so as to maintain a continuous (e.g., constant) flow of fluid to the microdialysis probe 30 and a continuous (e.g., constant) flow of fluid having exited the microdialysis probe 30 to the sensor module 50 over a selected period of time, e.g., one or several hours, or one or several days and nights.

In alternative or in addition, the control module 90 may be configured to control operation of the at least one fluid flow transport device 10 at least so as to convey the flow of fluid including at least one reagent so as to join with the at least one fluid flow path for flow of fluid to the sensor module 50, e.g., by controllably conveying the flow of fluid including at least one reagent into the at least one fluid flow conduit 60, so as to maintain a continuous (e.g., constant) flow of the fluid including at least one reagent over said selected period of time or over another period of time. The other period of time may be at least in part overlapping with the said selected period of time.

The at least one fluid flow transport device 10 may be configured to selectively convey a flow of calibration fluid so as to join with the at least one fluid flow path for flow of fluid to the sensor module 50 via the second fluid flow connector 80 while not conveying a flow of fluid to the microdialysis probe 30. Thereby, there may not be conveyed a flow of fluid having exited the microdialysis probe 30 to the sensor module 50 via the at least one fluid flow path for flow of fluid to the sensor module 50. This may be done in order to carry out a calibration process for the sensor module 50, and may be done based on at least one predefined trigger criterion - for example, it may be done if at least one trigger criterion is met. The at least one predefined trigger criterion may for example comprise: that a selected period of time of use of the microdialysis system 100 has elapsed, and/or that a selected period of time since the carrying out of a previous calibration process has elapsed. Determining or monitoring whether a selected period of time of use of the microdialysis system 100 and/or a selected period of time since the carrying out of a previous calibration process has elapsed may be carried out for example by the control module 90, or by some other entity which may be included in the microdialysis system 100. The calibration process for the sensor module 50 may be carried out automatically, for example on a condition that at least one of the at least one predefined trigger criterion is met. To that end, the control module 90 (or any other control module which may be comprised in the microdialysis system 100) may be configured to control operation of the at least one fluid flow transport device 10 at least such that the calibration process for the sensor module 50 is automatically carried out on a condition that at least one of the at least one predefined trigger criterion is met.

In accordance with the one or more embodiments of the present invention illustrated in Figure 1, the fluid flow connector 70, for joining flow of fluid including at least one reagent with the at least one fluid flow path for flow of fluid to the sensor module 50, and the fluid flow connector 80, for joining flow of calibration fluid with the at least one fluid flow path for flow of fluid to the sensor module 50, in the foregoing referred to as the first fluid flow connector and the second fluid flow connector, respectively, are different fluid flow connectors. This is however not required, and one fluid flow connector could be provided for joining flow of fluid including at least one reagent with the at least one fluid flow path for flow of fluid to the sensor module 50 as well as for joining flow of calibration fluid with the at least one fluid flow path for flow of fluid to the sensor module 50.

Further in accordance with the one or more embodiments of the present invention illustrated in Figure 1, the second fluid flow connector 80 is downstream the first fluid flow connector 70. Thus, in Figure 1, the joining of the flow of calibration fluid with the at least one fluid flow path for flow of fluid to the sensor module 50 is at a position downstream the joining of the flow of fluid including at least one reagent with the at least one fluid flow path for flow of fluid to the sensor module 50. However, in alternative, the first fluid flow connector 70 could be downstream the second fluid flow connector 80. That is, the joining of the flow of fluid including at least one reagent with the at least one fluid flow path for flow of fluid to the sensor module 50 could be at a position downstream the joining of the flow of calibration fluid with the at least one fluid flow path for flow of fluid to the sensor module 50.

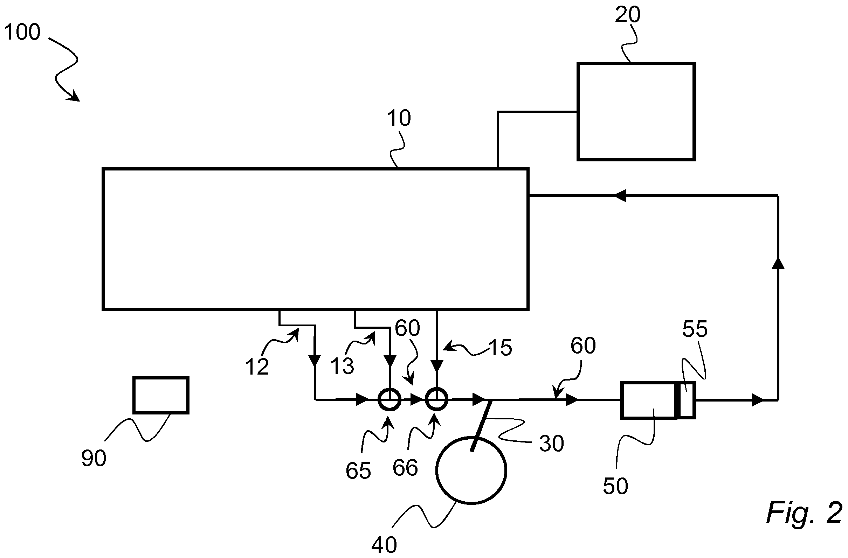

Figure 2 is a (very) schematic view of a microdialysis system 100 according to one or more embodiments of the present invention, for illustrating principles of one or more embodiments of the present invention. The microdialysis system 100 illustrated in Figure 2 is similar to the microdialysis system 100 illustrated in Figure 1, and the same reference numerals in Figures 1 and 2 indicate the same or similar components, having the same or similar function(s).

In accordance with the one or more embodiments of the present invention illustrated in Figure 2, and as illustrated in Figure 2, there is one fluid flow connector 65 for joining flow of fluid including at least one reagent with the at least one fluid flow path for flow of fluid to the sensor module 50 and for joining flow of calibration fluid with the at least one fluid flow path for flow of fluid to the sensor module 50. Thus, in accordance with the one or more embodiments of the present invention illustrated in Figure 2, the fluid flow connector for joining flow of fluid including at least one reagent with the at least one fluid flow path for flow of fluid to the sensor module 50 and the fluid flow connector for joining flow of calibration fluid with the at least one fluid flow path for flow of fluid to the sensor module 50 are the same, and are constituted by one fluid flow connector 65, instead of different fluid flow connectors as illustrated in Figure 1.

As illustrated in Figure 2, the fluid flow connector 65 may be in fluid communication with the analyzer 10 via fluid flow conduits 12 and 13. A flow of calibration fluid and a flow of fluid including at least one reagent may be selectively conveyed from the analyzer 10 via the fluid flow conduits 12 and 13. The flow of calibration fluid may be selectively conveyed from the analyzer 10 via the fluid flow conduit 12, and the flow of fluid including at least one reagent may be selectively conveyed from the analyzer 10 via the fluid flow conduit 13. In alternative, the flow of calibration fluid could be selectively conveyed from the analyzer 10 via the fluid flow conduit 13, and the flow of fluid including at least one reagent could be selectively conveyed from the analyzer 10 via the fluid flow conduit 12.

Further in accordance with the one or more embodiments of the present invention illustrated in Figure 2, and as illustrated in Figure 2, the at least one fluid flow transport device may for example be configured to selectively convey a flow of fluid to the microdialysis probe 30 via a fluid flow conduit 15, which may possibly be referred to as a perfusate fluid flow conduit 15, or physiological fluid flow conduit 15, similarly to the fluid flow conduit 15 illustrated in Figure 1. As illustrated in Figure 2, the analyzer 10 and the microdialysis probe 30 may be in fluid communication by means of the fluid flow conduit 15. The at least one fluid flow transport device may for example be configured to selectively convey a flow of physiological fluid to the microdialysis probe 30 via the fluid flow conduit 15 and a fluid flow connector 66. Thus, by means of the fluid flow conduit 15 and the fluid flow connector 66, a flow of physiological fluid to the microdialysis probe 30 may be joined with the at least one fluid flow path for flow of fluid to the sensor module 50. In accordance with the one or more embodiments of the present invention illustrated in Figure 2, and as illustrated in Figure 2, the microdialysis probe 30 is downstream the fluid flow connector 66.

The fluid flow connector 65 and/or the fluid flow connector 66 may be configured or arranged so as to not require any moving parts for the joining of the respective fluid flows with the at least one fluid flow path for flow of fluid to the sensor module 50. That is, any moving parts of or in the fluid flow connector 65 and/or the fluid flow connector 66 for the joining of the respective fluid flows with the at least one fluid flow path for flow of fluid to the sensor module 50 may not be required. To that end, the fluid flow connector 65 and/or the fluid flow connector 66 may for example comprise a valveless fluid flow connector.

In accordance with the one or more embodiments of the present invention illustrated in Figure 2, the joining of the flow of fluid including at least one reagent and the flow of calibration fluid with the at least one fluid flow path for flow of fluid to the sensor module 50 is in a position in the at least one fluid flow path that is upstream the position in the at least one fluid flow path in which the flow of (e.g., physiological) fluid to the microdialysis probe 30 is joined with the at least one fluid flow path. However, it is to be understood that the positions along the at least one fluid flow path in a flow direction towards the sensor module 50 in which the respective ones of the flow of fluid including at least one reagent, the flow of calibration fluid, and the flow of (e.g., physiological) fluid to the microdialysis probe 30 are joined with the at least one fluid flow path may be in any order.

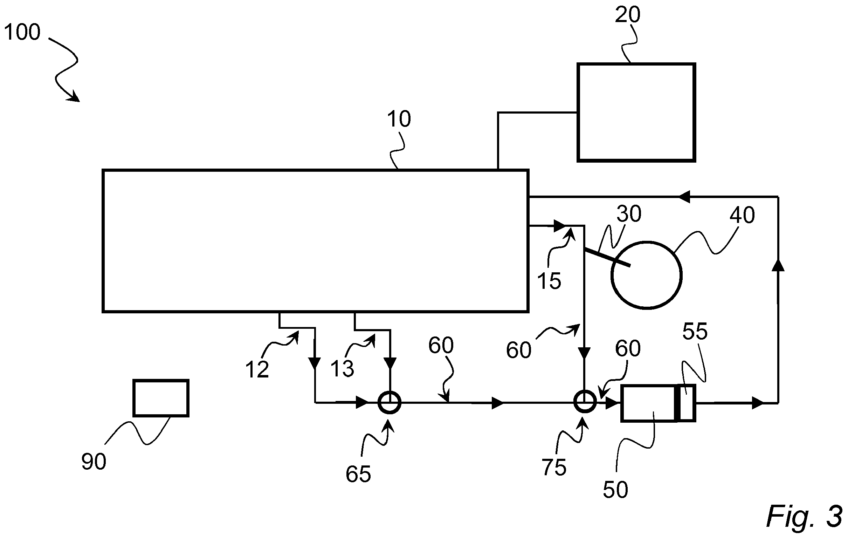

Figure 3 is a (very) schematic view of a microdialysis system 100 according to one or more embodiments of the present invention. The microdialysis system 100 illustrated in Figure 3 is similar to the microdialysis system 100 illustrated in Figure 2, and the same reference numerals in Figures 2 and 3 indicate the same or similar components, having the same or similar function(s).

In accordance with the one or more embodiments of the present invention illustrated in Figure 3, and as illustrated in Figure 3, the at least one fluid flow transport device may for example be configured to selectively convey a flow of fluid to the microdialysis probe 30 via a fluid flow conduit 15, which may possibly be referred to as a perfusate fluid flow conduit 15, or physiological fluid flow conduit 15, similarly to the fluid flow conduit 15 illustrated in Figure 1. As illustrated in Figure 3, the analyzer 10 and the microdialysis probe 30 may be in fluid communication by means of the fluid flow conduit 15.

Further in accordance with the one or more embodiments of the present invention illustrated in Figure 3, the at least one fluid flow transport device may be configured to selectively convey the flow of fluid having exited the microdialysis probe 30 so as to join with the at least one fluid flow path for flow of fluid to the sensor module 50 via a fluid flow connector 75 for joining flow of fluid having exited the microdialysis probe 30 with the at least one fluid flow path for flow of fluid to the sensor module 50. As illustrated in Figure 3, the fluid flow connector 75 may be arranged downstream the microdialysis probe 30 and upstream the sensor module 50.

The fluid flow connector 75 for joining flow of fluid having exited the microdialysis probe 30 with the at least one fluid flow path for flow of fluid to the sensor module 50 may be configured so as to permit joining of the flow of fluid having exited the microdialysis probe 30 with the at least one fluid flow path for flow of fluid to the sensor module 50 without requiring any moving parts. That is, any moving parts of or in the fluid flow connector 75 for joining flow of fluid having exited the microdialysis probe 30 with the at least one fluid flow path for flow of fluid to the sensor module 50 may not be required. To that end, the fluid flow connector 75 may for example comprise a valveless fluid flow connector.

Similar to in the microdialysis system 100 illustrated in Figures 1 and 2, the at least one fluid flow path for flow of fluid to the sensor module 50 in the microdialysis system 100 illustrated in Figure 3 comprises at least one fluid flow conduit 60. In accordance with the one or more embodiments of the present invention illustrated in Figure 3, the at least one fluid flow conduit 60 comprises the fluid flow connector 75 for joining flow of fluid having exited the microdialysis probe 30 with the at least one fluid flow path for flow of fluid to the sensor module 50. The at least one fluid flow transport device may be configured to selectively convey the flow of fluid having exited the microdialysis probe 30 into the at least one fluid flow conduit 60 via the fluid flow connector 75 for joining flow of fluid having exited the microdialysis probe 30 with the at least one fluid flow path for flow of fluid to the sensor module 50.

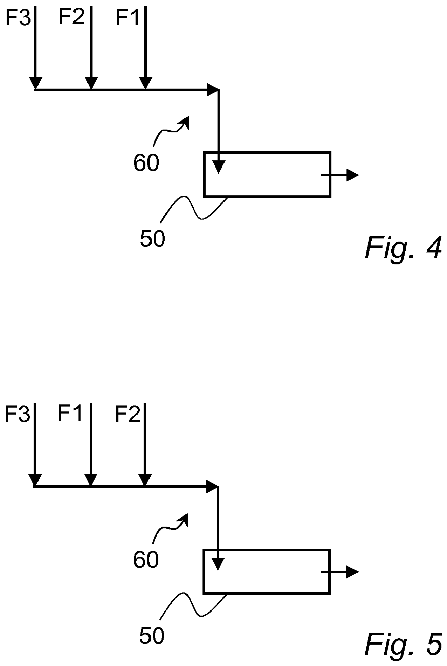

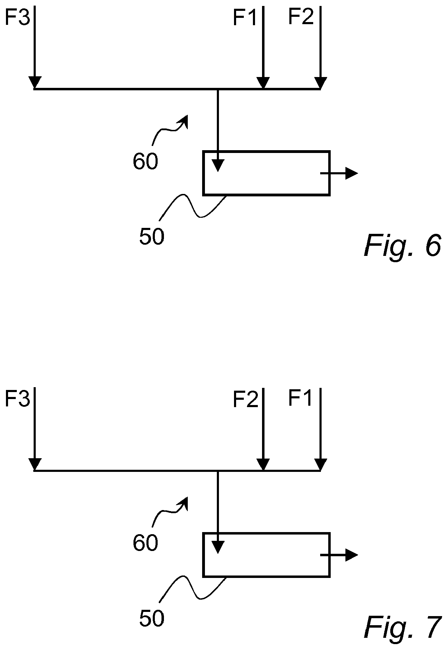

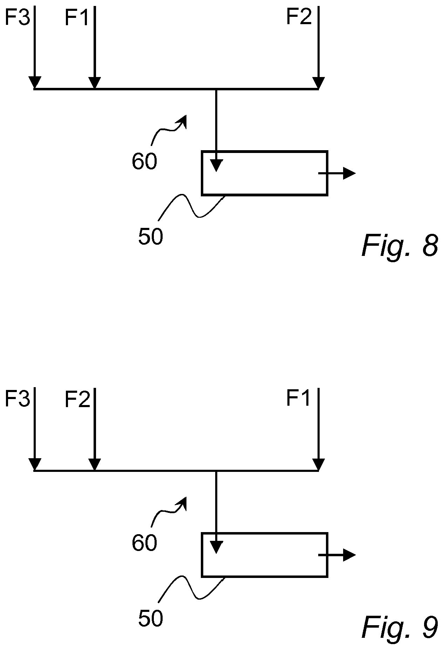

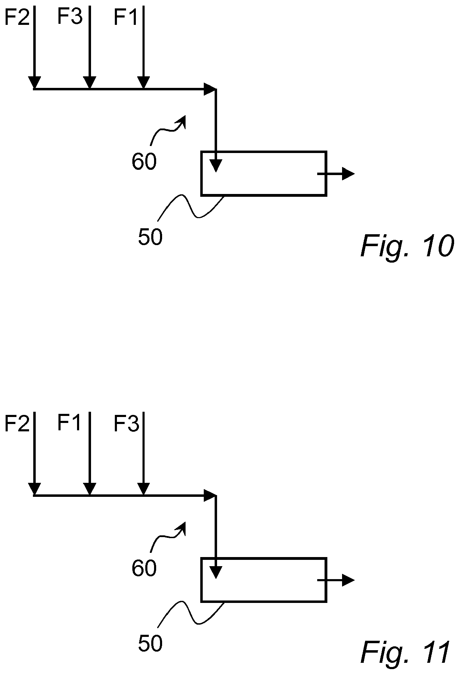

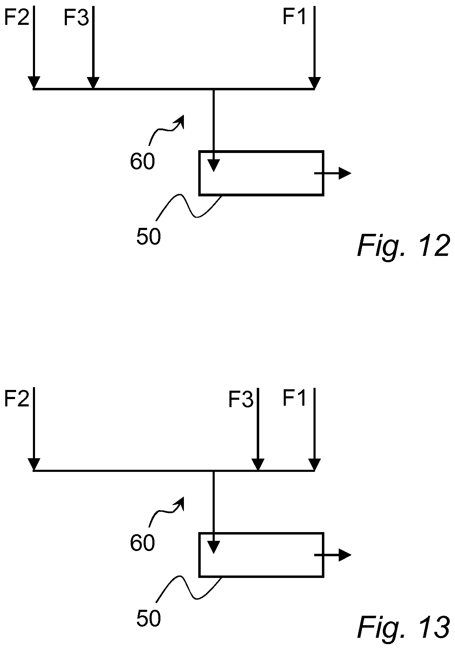

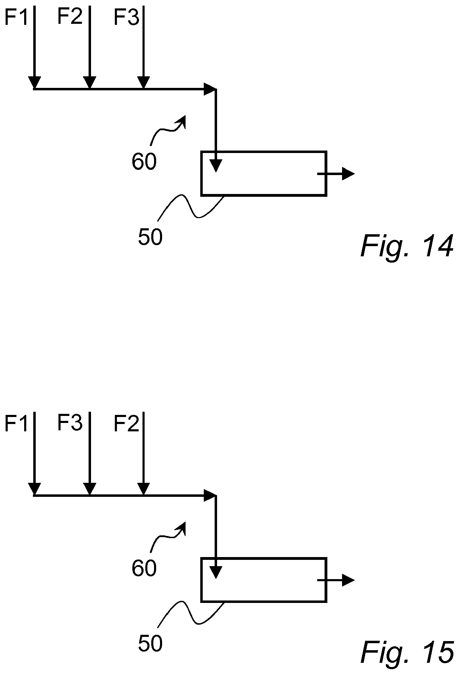

Some ways of how the flow of fluid having exited the microdialysis probe 30, the flow of fluid including at least one reagent, and the flow of calibration fluid may be arranged in relation to each other and in relation to the sensor module 50 in the microdialysis system 100 have been illustrated in Figures 1 to 3. It is however to be understood that the relative arrangements of the respective ones of the above-mentioned flows illustrated in Figures 1 to 3 are exemplifying, and not limiting, and that variations are possible. Such variations are illustrated in Figures 4 to 15.

Figures 4 to 15 schematically illustrate relative arrangements of the respective ones of the above-mentioned flows with respect to the sensor module 50 in accordance with embodiments of the present invention (of which some are in accordance with the one or more embodiments of the present invention illustrated in Figure 1 to 3). In Figures 4 to 15, the flow of fluid having exited the microdialysis probe 30, the flow of fluid including at least one reagent, and the flow of calibration fluid are denoted by F1, F2, and F3, respectively, and the sensor module is denoted by reference numeral 50, as in Figures 1 to 3. In Figures 4 to 15, the arrows indicate flow directions, and the reference numeral 60 indicates the at least one fluid flow path (e.g., comprising at least one fluid flow conduit) for flow of fluid to the sensor module 50.

In conclusion, a microdialysis system is disclosed, comprising a microdialysis probe insertable or inserted into tissue of a subject, a sensor module configured to receive at least one fluid flow and sense at least one substance in the received at least one fluid flow, at least one fluid flow transport device configured to selectively convey a flow of fluid to the microdialysis probe, and at least one fluid flow path for flow of fluid to the sensor module. The at least one fluid flow transport device is configured to selectively convey a flow of fluid including at least one reagent so as to join with the at least one fluid flow path for flow of fluid to the sensor module via at least one fluid flow connector for joining flow of fluid including at least one reagent with the at least one fluid flow path for flow of fluid to the sensor module. The at least one fluid flow transport device is further configured to selectively convey a flow of calibration fluid so as to join with the at least one fluid flow path for flow of fluid to the sensor module via at least one fluid flow connector for joining flow of calibration fluid with the at least one fluid flow path for flow of fluid to the sensor module. Each of the at least one fluid flow connector is arranged and/or configured so as permit the joining of the respective flows with the at least one fluid flow path for flow of fluid to the sensor module without requiring any moving parts, e.g., by being implemented as one or more valveless fluid flow connectors.

While the present invention has been illustrated in the appended drawings and the foregoing description, such illustration is to be considered illustrative or exemplifying and not restrictive; the present invention is not limited to the disclosed embodiments. Other variations to the disclosed embodiments can be understood and effected by those skilled in the art in practicing the claimed invention, from a study of the drawings, the disclosure, and the appended claims. In the appended claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A microdialysis system (100), comprising:
a microdialysis probe (30) insertable or inserted into tissue of a subject (40), wherein the microdialysis probe is configured so that it can receive a fluid flow and so that it can output a fluid flow;
a sensor module (50) configured to receive at least one fluid flow and sense at least one substance in the received at least one fluid flow;
at least one fluid flow transport device configured to selectively convey a flow of fluid to the microdialysis probe; and
at least one fluid flow path (60) for flow of fluid to the sensor module;
wherein the at least one fluid flow transport device is further configured to convey a flow of fluid having exited the microdialysis probe into, or so as to join with, the at least one fluid flow path (60) for flow of fluid to the sensor module;
wherein the at least one fluid flow transport device is further configured to selectively convey a flow of fluid including at least one reagent so as to join with the at least one fluid flow path for flow of fluid to the sensor module via at least one fluid flow connector (65; 70) for joining flow of fluid including at least one reagent with the at least one fluid flow path for flow of fluid to the sensor module;
wherein the at least one fluid flow transport device is further configured to selectively convey a flow of calibration fluid so as to join with the at least one fluid flow path for flow of fluid to the sensor module via at least one fluid flow connector (65; 80) for joining flow of calibration fluid with the at least one fluid flow path for flow of fluid to the sensor module;
wherein each of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module is configured so as to permit joining of the flow of fluid including at least one reagent and the flow of calibration fluid, respectively, with the at least one fluid flow path for flow of fluid to the sensor module without requiring any moving parts.

2. A microdialysis system according to claim 1, wherein the at least one fluid flow path for flow of fluid to the sensor module comprises at least one fluid flow conduit (60) for conveying of fluid to the sensor module, the at least one fluid flow conduit comprising the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module, each of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module being arranged downstream the microdialysis probe and upstream the sensor module;
wherein the at least one fluid flow transport device is configured to selectively convey the flow of fluid including at least one reagent into the at least one fluid flow conduit via the at least one fluid flow connector for joining flow of fluid including at least one reagent with the at least one fluid flow path for flow of fluid to the sensor module;
wherein the at least one fluid flow transport device is configured to selectively convey the flow of calibration fluid into the at least one fluid flow conduit via the at least one fluid flow connector for joining flow of calibration fluid with the at least one fluid flow path for flow of fluid to the sensor module.

3. A microdialysis system according to any claim 1 or 2, wherein at least one of the at least one fluid flow connector for joining flow of fluid including at least one reagent with the at least one fluid flow path for flow of fluid to the sensor module and the at least one fluid flow connector for joining flow of calibration fluid with the at least one fluid flow path for flow of fluid to the sensor module comprises a valveless fluid flow connector.

4. A microdialysis system according to any one of the preceding claims, wherein the at least one fluid flow transport device is further configured to selectively convey the flow of fluid having exited the microdialysis probe so as to join with the at least one fluid flow path for flow of fluid to the sensor module via at least one fluid flow connector (75) for joining flow of fluid having exited the microdialysis probe with the at least one fluid flow path for flow of fluid to the sensor module, the at least one fluid flow connector for joining flow of fluid having exited the microdialysis probe with the at least one fluid flow path for flow of fluid to the sensor module being arranged downstream the microdialysis probe and upstream the sensor module, and wherein the at least one fluid flow connector for joining flow of fluid having exited the microdialysis probe with the at least one fluid flow path for flow of fluid to the sensor module is configured so as to permit joining of the flow of fluid having exited the microdialysis probe with the at least one fluid flow path for flow of fluid to the sensor module without requiring any moving parts.

5. A microdialysis system according to any one of the preceding claims, wherein the at least one fluid flow transport device is further configured to convey the flow of fluid having exited the microdialysis probe into, or so as to join with, the at least one fluid flow path for flow of fluid to the sensor module at a position in the at least one fluid flow path for flow of fluid to the sensor module that is downstream a position in the at least one fluid flow path for flow of fluid to the sensor module in which the flow of fluid including at least one reagent is joined with the at least one fluid flow path for flow of fluid to the sensor module and/or a position in the at least one fluid flow path for flow of fluid to the sensor module in which the flow of calibration fluid is joined with the at least one fluid flow path for flow of fluid to the sensor module.

6. A microdialysis system according to any one of claims 1-4, wherein the at least one fluid flow transport device is further configured to selectively convey the flow of fluid including at least one reagent so as to join with the at least one fluid flow path for flow of fluid to the sensor module at a position in the at least one fluid flow path for flow of fluid to the sensor module that is downstream a position in the at least one fluid flow path for flow of fluid to the sensor module in which the flow of fluid having exited the microdialysis probe is joined with the at least one fluid flow path for flow of fluid to the sensor module and/or a position in the at least one fluid flow path for flow of fluid to the sensor module in which the flow of calibration fluid is joined with the at least one fluid flow path for flow of fluid to the sensor module.

7. A microdialysis system according to any one of claims 1-4, wherein the at least one fluid flow transport device is further configured to selectively convey the flow of calibration fluid so as to join with the at least one fluid flow path for flow of fluid to the sensor module that is downstream a position in the at least one fluid flow path for flow of fluid to the sensor module in which the flow of fluid having exited the microdialysis probe is joined with the at least one fluid flow path for flow of fluid to the sensor module and/or a position in the at least one fluid flow path for flow of fluid to the sensor module in which the flow of fluid including at least one reagent is/are joined with the at least one fluid flow path for flow of fluid to the sensor module is joined with the at least one fluid flow path for flow of fluid to the sensor module.

8. A microdialysis system according to any one of the preceding claims, wherein the at least one fluid flow transport device is further configured to join two of the flow of fluid having exited the microdialysis probe, the flow of fluid including at least one reagent, and the flow of calibration fluid so as to obtain a combined fluid flow, and wherein the at least one fluid flow transport device is further configured to join the third one of the flow of fluid having exited the microdialysis probe, the flow of fluid including at least one reagent, and the flow of calibration fluid with the combined fluid flow at a position in the at least one fluid flow path for flow of fluid to the sensor module that is downstream the microdialysis probe.

9. A microdialysis system according to any one of the preceding claims, wherein the least one fluid flow transport device is configured to controllably convey the flow of fluid to the microdialysis probe and convey the flow of fluid having exited the microdialysis probe to the sensor module, at least with respect to a flow rate of the flow of fluid to the microdialysis probe and a flow rate of the flow of fluid having exited the microdialysis probe to the sensor module, respectively, the microdialysis system further comprising:
at least one control module (90) configured to control operation of the at least one fluid flow transport device at least so as to:
convey the flow of fluid to the microdialysis probe and convey the flow of fluid having exited the microdialysis probe to the sensor module so as to maintain a continuous flow of fluid to the microdialysis probe and a continuous flow of fluid having exited the microdialysis probe to the sensor module over a selected period of time;
wherein the at least one fluid flow transport device is further configured to controllably convey the flow of fluid including at least one reagent so as to join with the at least one fluid flow path for flow of fluid to the sensor module, at least with respect to a flow rate of the flow of fluid including at least one reagent, wherein the at least one control module is further configured to control operation of the at least one fluid flow transport device at least so as to:
convey the flow of fluid including at least one reagent so as to join with the at least one fluid flow path for flow of fluid the sensor module so as to maintain a continuous flow of the fluid including at least one reagent over said selected period of time or over another period of time.

10. A microdialysis system according to any one of the preceding claims, wherein, based on at least one predefined trigger criterion, the at least one fluid flow transport device is configured to selectively convey a flow of calibration fluid so as to join with the at least one fluid flow path for flow of fluid to the sensor module via the at least one fluid flow connector for joining flow of calibration fluid with the at least one fluid flow path for flow of fluid to the sensor module while not conveying a flow of fluid to the microdialysis probe, thereby not conveying a flow of fluid having exited the microdialysis probe to the sensor module via the at least one fluid flow path for flow of fluid to the sensor module, in order to carry out a calibration process for the sensor module;
wherein the at least one predefined trigger criterion comprises at least one of:
a selected period of time of use of the microdialysis system has elapsed; or
a selected period of time since the carrying out of a previous calibration process has elapsed.

11. A microdialysis system according to any one of the preceding claims, wherein the at least one fluid flow connector (65) for joining flow of fluid including at least one reagent with the at least one fluid flow path for flow of fluid to the sensor module and the at least one fluid flow connector (65) for joining flow of calibration fluid with the at least one fluid flow path for flow of fluid to the sensor module are the same.

12. A microdialysis system according to any one of claims 1-10, wherein the at least one fluid flow connector (70) for joining flow of fluid including at least one reagent with the at least one fluid flow path for flow of fluid to the sensor module and the at least one fluid flow connector (80) for joining flow of calibration fluid with the at least one fluid flow path for flow of fluid to the sensor module are different, and wherein the at least one fluid flow connector (80) for joining flow of calibration fluid with the at least one fluid flow path for flow of fluid to the sensor module is downstream the at least one fluid flow connector (70) for joining flow of fluid including at least one reagent with the at least one fluid flow path for flow of fluid to the sensor module.

13. A microdialysis system according to any one of claims 1-10, wherein the at least one fluid flow connector (70) for joining flow of fluid including at least one reagent with the at least one fluid flow path for flow of fluid to the sensor module and the at least one fluid flow connector (80) for joining flow of calibration fluid with the at least one fluid flow path for flow of fluid to the sensor module are different, and wherein the at least one fluid flow connector for joining flow of fluid including at least one reagent with the at least one fluid flow path for flow of fluid to the sensor module is downstream the at least one fluid flow connector for joining flow of calibration fluid with the at least one fluid flow path for flow of fluid to the sensor module.

14. A microdialysis system according to any one of the preceding claims, wherein the sensor module is configured to sense at least one of glucose, lactate, or pyruvate.

15. A microdialysis system according to any one of the preceding claims, wherein the reagent includes at least one of phosphate, phosphoric acid, or one or more enzymes including lactate oxidase, glucose oxidase, or pyruvate oxidase.
